# EUROPÄISCHE PATENTANMELDUNG

(11) **EP 0 998 873 A2**
(43) Veröffentlichungstag der Anmeldung: **10.05.2000**
(21) Anmeldenummer: 99120431.4
(22) Anmeldetag: 14.10.1999
(51) Int. Cl.: A61B 3/12

(54) **Verfahren und Vorrichtung zum Fokussieren eines Lichtstromes**

(30) Priorität: 22.01.1999 CH 12099; 07.11.1998 CH 223598
(71) Anmelder: GRIESHABER & CO. AG SCHAFFHAUSEN, CH-8203 Schaffhausen (CH)
(72) Erfinder: Grieshaber, Hans R., 8200 Schaffhausen (CH); Vogel, Urs, 8200 Schaffhausen (CH)
(74) Vertreter: Althoff, Gerhard

(57) **Zusammenfassung**

Es wird eine aus mehreren Funktionselementen bestehende Vorrichtung für die Aussen- und/oder Innenbeleuchtung eines Auges vorgeschlagen, wobei die Vorrichtung entweder als Baueinheit (3) in das Gehäuse (1) einer ophthalmologischen Einrichtung (100) einschiebbar oder aber als eine eigenständige Baueinheit ausgebildet und verwendbar ist.

Die Vorrichtung (30) umfasst mindestens einen Körper (35;35') mit zugeordneter Lichtquelle (40), von welcher der abgestrahlte Lichtstrom durch einen ersten Lichtkanal (L1;L1') und darin angeordneter erster optischer Linse (47;47') über ein die Wärme mindestens teilweise auskoppelndes Bauteil (43;43') einem abgewinkelt zum ersten Lichtkanal (L1;L1) orientierten zweiten Lichtkanal (L2;L2') und über eine darin angeordnete zweite optische Linse (48;48') auf mindestens eine in die Vorrichtung (30) einsteckbare Beleuchtungssonde (10; 10') fokussiert wird.

## Beschreibung

Die Erfindung bezieht sich auf ein Verfahren sowie auf eine Vorrichtung zum Fokussieren eines von einer Lichtquelle abgestrahlten Lichtstromes, welcher über mindestens eine mit einem Lichtkanal in Verbindung stehende optische Linse auf einen Lichtleiter, insbesondere auf einen als ophthalmoskopische Beleuchtungssonde ausgebildeten Lichtleiter fokussiert wird.

Bei Untersuchungen und/oder Operationen an einem Auge, insbesondere bei der Glaskörperchirurgie ist die Verwendung einer Beleuchtungssonde, vorzugsweise in Form eines Lichtleiters aus Glasfasern oder Kunststofffasern an sich bekannt. Die einzelne Beleuchtungssonde ist dabei beispielsweise an eine mit einer entsprechenden Lichtquelle sowie entsprechend angeordneten optischen Elementen versehene Vorrichtung angeschlossen. Bei den bekannten Vorrichtungen besteht die Möglichkeit, dass nach relativ kurzer Betriebszeit der Lichtquelle eine ausreichende Beleuchtung des Glaskörperhohlraums nicht mehr oder nur noch bedingt möglich oder aber die Lichtquelle defekt ist. Ein weiterer Nachteil der bekannten Vorrichtungen besteht in den unerwünschten Nebeneffekten, beispielsweise in Form einer relativ hohen Wärmeentwicklung der Lichtquelle.

Aufgabe der Erfindung ist es, ein Verfahren sowie eine Vorrichtung zur Durchführung des Verfahrens zu schaffen, mittels derer bei ab- und zunehmender Beleuchtung mit möglichst geringer Wärmeentwicklung austrittsseitig ein weitgehend gleichbleibender und auf das Anschlusselement, beispielsweise auf eine Beleuchtungssonde übertragbarer Lichtkegeldurchmesser erreicht wird.

Zur Lösung der Aufgabe hinsichtlich des Verfahrens führt, dass der Lichtstrom von einer ersten optischen Linse durch einen ersten Lichtkanal auf ein zugeordnetes und die Wärme mindestens teilweise auskoppelndes Bauteil geleitet und von diesem die sichtbare Strahlung in einen abgewinkelt zu dem ersten Lichtkanal orientierten zweiten Lichtkanal umgelenkt wird, wobei die sichtbare Strahlung in dem zweiten Lichtkanal entweder zuerst eine zur wahlweisen Regulierung der Lichtintensität ausgebildete Blendscheibe durchdringend auf eine zweite optische Linse geleitet und von dieser auf die Beleuchtungssonde fokussiert wird beziehungsweise zuerst auf die zweite optische Linse und von dieser die in Richtung der sichtbaren Strahlung nachgeschaltete Blendscheibe durchdringend auf die Beleuchtungssonde fokussiert wird.

Weitere erfindungswesentliche Merkmale hinsichtlich des Verfahrens ergeben sich aus der nachstehenden Beschreibung und den Patentansprüchen 2 bis 6.

Die erfindungsgemässe Einrichtung gemäss dem Oberbegriff des Anspruchs 7 ist gekennzeichnet durch mindestens einen der Lichtquelle zugeordneten Körper mit in Richtung des abgestrahlten Lichtstromes orientiertem ersten Lichtkanal und abgewinkelt dazu angeordnetem zweiten Lichtkanal, und dass eingangsseitig des ersten Lichtkanals eine erste optische Linse und ausgangsseitig ein die sichtbare Strahlung in den zweiten Lichtkanal umlenkendes Bauteil angeordnet ist, wobei in dem zweiten Lichtkanal in Richtung der umgelenkten Strahlung gesehen entweder zuerst eine zur Regulierung der Lichtintensität ausgebildete sowie in bezug auf den zweiten Lichtkanal einstellbare Blendscheibe und dieser nachgeordnet eine die umgelenkte Strahlung fokussierende zweite optische Linse oder umgekehrt in Richtung der umgelenkten Strahlung gesehen zuerst die zweite Linse und dieser nachgeordnet die Blendscheibe angeordnet ist.

Weitere erfindungswesentliche Merkmale hinsichtlich der Vorrichtung ergeben sich aus der nachstehenden Beschreibung und den weiteren Patentansprüchen 8 bis 21.

Mit der erfindungsgemässen Vorrichtung besteht die Möglichkeit, dass mit einer einzigen zentralen Lichtquelle beispielsweise zwei voneinander getrennte Lichtkanäle beaufschlagbar sind, welche beispielsweise zum Anschliessen einer ersten ophthalmoskopischen Beleuchtungssonde für eine Grundbeleuchtung sowie zum Anschliessen einer zweiten ophthalmoskopischen Beleuchtungssonde für eine Arbeitsbeleuchtung ausgebildet sind. Bei einem bevorzugten Ausführungsbeispiel besteht zudem die Möglichkeit, dass die an den beiden Lichtausgängen anliegenden und fokussierten Lichtströme unter Zwischenschaltung einer in jedem Strahlengang angeordneten Blendscheibe unabhängig voneinander regelbar sind. Eine weitere bevorzugte Variante besteht in der Zuschaltung eines an der einen Blendscheibe angeordneten Farbfilters, beispielsweise eines Grünfilters und an der anderen Blendscheibe angeordneten Fluoreszenzfilters, wobei die Zuschaltung der beiden Filter miteinander oder aber getrennt voneinander erfolgen kann. Die beiden Blendscheiben sind weiterhin an der Oberfläche derart beschichtet, dass diese in Abhängigkeit der Drehrichtung sowie Position eine weitgehend gleichmässige ab- oder zunehmende Beleuchtung bei gleichbleibendem Lichtkegeldurchmesser gewährleisten. Die beiden Blendscheiben ermöglichen jeweils eine maximale Lichtdurchlässigkeit von 100% sowie anschliessende gleichmässige Abblendbereiche bis zu einer weitgehend vollständigen Lichtundurchlässigkeit.

Weitere Merkmale der Erfindung ergeben sich aus der nachstehenden Beschreibung in Verbindung mit der Zeichnung und den einzelnen Patentansprüchen.

Ein Ausführungsbeispiel der Erfindung wird nachstehend anhand der Zeichnung beschrieben. Es zeigt:
- **Fig.1**: eine in räumlicher Ansicht dargestellte ophthalmologische Einrichtung mit darin angeordneter Beleuchtungseinheit und mindestens einer daran anschliessbaren ophthalmoskopischen Beleuchtungssonde;
- **Fig.2**: die in räumlicher Ansicht dargestellte Einrichtung gemäss Fig.1 mit einer teilweise herausgezogen dargestellten Beleuchtungseinheit;
- **Fig.3**: ein schematisch und im Schnitt dargestelltes Gehäuse mit darin angeordneten Funktionselementen für die Beleuchtungseinheit gemäss Fig.2;
- **Fig.4**: eine schematisch dargestellte Vorrichtung mit optischen Funktionselementen für die Beleuchtungseinheit gemäss Fig.2;
- **Fig.5**: ein erstes Teilstück eines schematisch dargestellten Antriebs für zwei jeweils um eine Achse drehbare Blendscheiben der Vorrichtung gemäss Fig.4;
- **Fig.6**: ein zweites Teilstück des Antriebs für die beiden Blendscheiben der Vorrichtung gemäss Fig.4;
- **Fig.7**: den gemäss der in Fig.5 eingezeichneten Linie VII-VII im Schnitt dargestellten Antrieb für die beiden Blendscheiben;
- **Fig.8**: eine gemäss der Linie VIII-VIII in Fig.7 im Schnitt dargestellte Halterung für einen an der Blendscheibe angeordneten Filter;
- **Fig.9**: die in Form eines Rohlings in Draufsicht dargestellte Blendscheibe; und
- **Fig.10**: die in Draufsicht dargestellte Blendscheibe gemäss Fig.9 mit einer Anzahl daran angeordneter Abblendflächen.

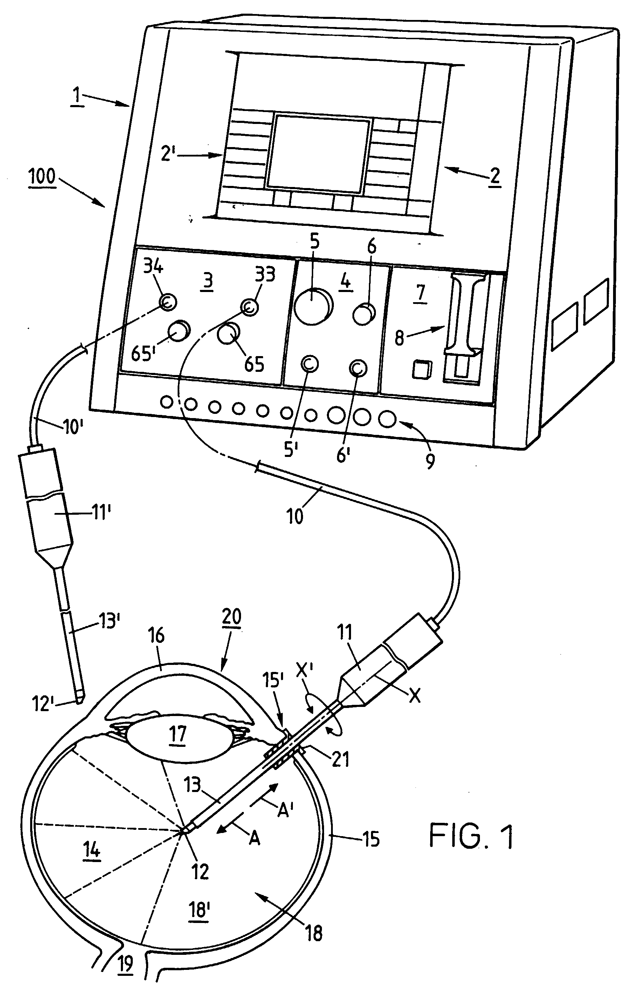

Fig.1 zeigt eine in perspektivischer Ansicht dargestellte Einrichtung, insbesondere eine zur Verwendung bei mikrochirurgischen Eingriffen am Auge eines Lebewesens ausgebildete ophthalmologische Einrichtung 100 zum Anschliessen entsprechend ausgebildeter Instrumente. Die schematisch dargestellte Einrichtung 100 umfasst im dargestellten Ausführungsbeispiel mehrere in einem Gehäuse 1 angeordnete und für den jeweiligen operativen Eingriff erforderliche Funktionseinheiten 3,4 und 7,8 sowie eine an der Frontseite oberhalb der Funktionseinheiten angeordnete Anzeigeeinheit 2. Die Anzeigeeinheit 2 ist mit mehreren nicht näher dargestellten und als Tasten, Druckknöpfe oder dergleichen ausgebildeten Bedienungselementen 2' versehen. Die Funktionseinheiten 3 und 4 sowie 7,8 sind beispielsweise als nicht näher dargestellte Einschübe ausgebildet, welche auswechselbar in das Gehäuse 1 einschiebbar und wieder herausziehbar sind. Weiterhin erkennt man eine unterhalb der einzelnen Funktionseinheiten 3 und 4 sowie 7,8 angeordnete und mit mehreren Anschlüssen versehene Steckerleiste 9 für weitere nicht dargestellte Instrumente.

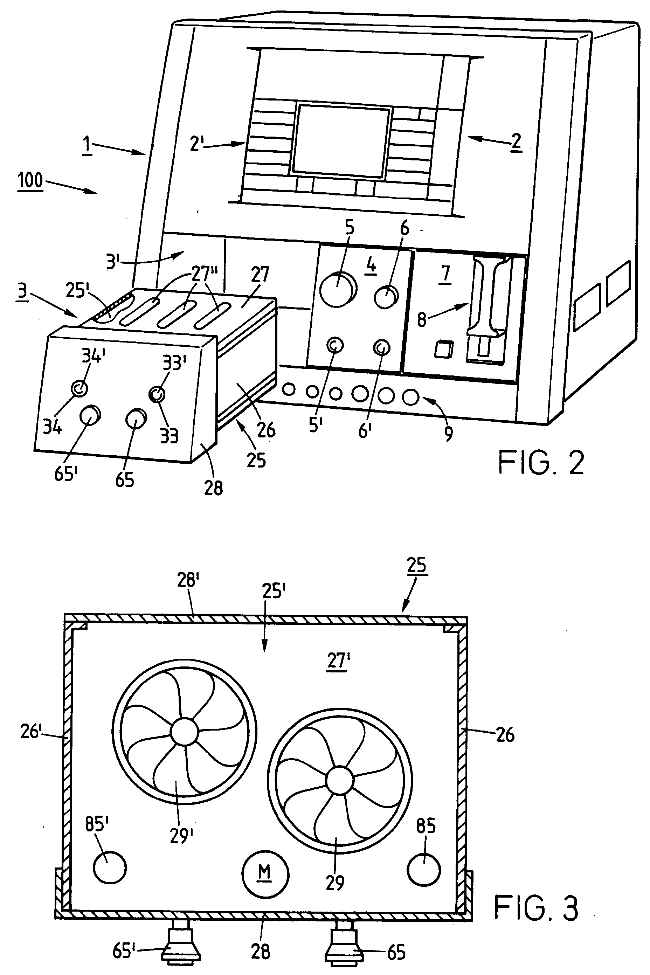

Die erste Funktionseinheit 3 wird nachstehend als Beleuchtungseinheit 3 bezeichnet, welche im dargestellten Ausführungsbeispiel mit zwei stirnseitig angeordneten Einstellknöpfen 65 und 65' sowie mit zwei jeweils zum Anschliessen einer ophthalmoskopischen Beleuchtungssonde ausgebildete Anschlussbuchsen 33 und 34 versehen ist. Die Beleuchtungseinheit 3 sowie die einzelnen Funktionselemente derselben werden später in Verbindung mit den Figuren 2 bis 10 im einzelnen beschrieben.

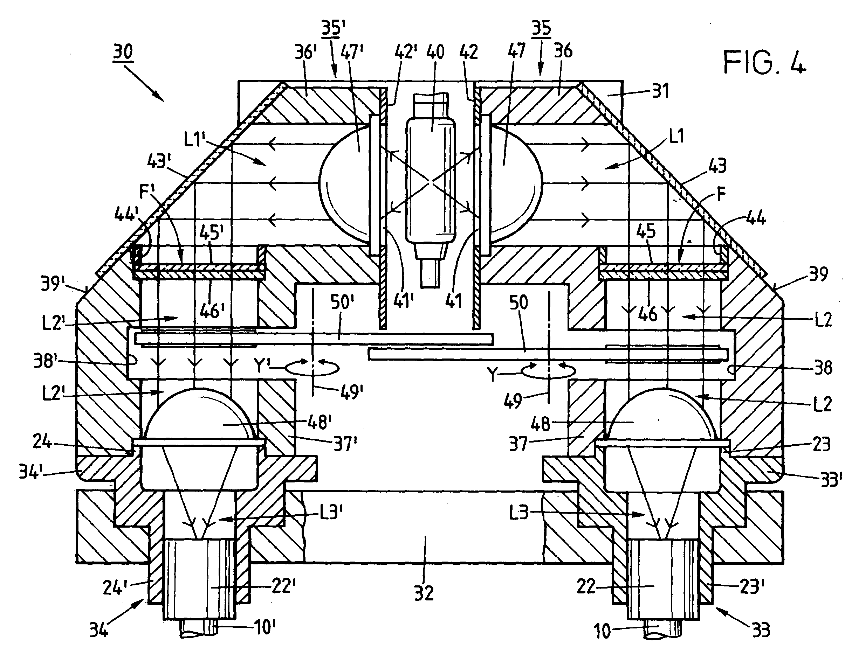

Bei dem in Fig.1 dargestellten Ausführungsbeispiel der Beleuchtungseinheit 3 ist in die erste Anschlussbuchse 33 eine erste ophthalmoskopische Beleuchtungssonde 10 und in die zweite Anschlussbuchse 34 eine zweite ophthalmoskopische Beleuchtungssonde 10' eingesteckt. Die beiden schematisch dargestellten Beleuchtungssonden 10,10' sind an dem einen Ende jeweils mit einem in die jeweilige Anschlussbuchse 33,34 einsteckbaren Kontaktelement 22,22' (Fig.4) versehen. In eingestecktem Zustand steht die einzelne Beleuchtungssonde 10 und 10' mit der Beleuchtungseinheit 3 in Wirkverbindung. Die beiden Beleuchtungssonden 10 und 10' können jeweils in Form eines Lichtleiters aus Glasfasern oder Kunststofffasern oder dergleichen hergestellt sein.

Fig.1 zeigt weiterhin ein in grösserem Massstab sowie als Horizontalschnitt dargestelltes Auge 20 und man erkennt die Lederhaut 15 (SKLERA), die Hornhaut 16 (CORNEA), die Linse 17 (LENS), den Glaskörper 18 mit dem Hohlraum 18', das in der Gesamtheit mit 19 bezeichnete Sehnervenbündel (OPTIKUS) sowie die mit dem distalen Ende in den Glaskörperhohlraum 18' eingeführte erste Beleuchtungssonde 10. Die zweite Beleuchtungssonde 10' ist, wie in Fig.1 schematisch dargestellt, im Abstand zu dem Auge 20 angeordnet.

Bei einer Variante besteht jedoch auch die Möglichkeit, dass beispielsweise die eine Beleuchtungssonde 10 als Grundbeleuchtung und die andere Beleuchtungssonde 10' als Arbeitsbeleuchtung oder umgekehrt verwendet wird. An der Beleuchtungssonde 10 und 10' ist jeweils ein als Handgriff ausgebildetes Gehäuse 11,11' und daran eine Hohlnadel 13,13' angeordnet. Die Beleuchtungssonde 10,10' durchdringt in axialer Richtung gesehen das Gehäuse 11,11' sowie die Hohlnadel 13,13' und ist am vorderen lichtaustrittsseitigen Ende 12 und 12' vorzugsweise zur Erreichung eines möglichst grossen Lichtkegels 14 ausgebildet. Das Gehäuse 11,11' ist im dargestellten Ausführungsbeispiel mittels der daran angeordneten Hohlnadel 13,13' durch eine im Bereich der Pars plana 15' eingesetzte Kanüle 21 in den Glaskörperhohlraum 18' eingeführt und kann um die Längsachse X in Pfeilrichtung X' gedreht sowie in axialer Richtung relativ zu der Sklera 15 des Auges 20 in Pfeilrichtung A oder A' verschoben werden. Die beiden Beleuchtungssonden 10 und 10' sowie die einzelnen damit verbundenen Elemente sind nicht Gegenstand dieser Erfindung und werden deshalb nicht näher beschrieben.

Die zweite Funktionseinheit 4 der Einrichtung 100 wird nachstehend als Druckeinheit 4 bezeichnet, welche im dargestellten Ausführungsbeispiel mit einer ersten Anschlussbuchse 5' sowie einem Einstellknopf 5 zum Regulieren des intraokularen Druckes (IOP) während des operativen Eingriffs versehen ist. Weiterhin kann die Druckeinheit 4 mit einer zweiten Anschlussbuchse 6' und einem Einstellknopf 6 versehen sein, mittels welcher eine nicht näher dargestellte Visko-Injektionseinrichtung aktivierbar ist.

Die dritte Funktionseinheit 7 der Einrichtung 100 wird nachstehend als Irrigations- und Aspirationssystem 7 bezeichnet. Das Irrigations- und Aspirationssystem 7 umfasst eine mit entsprechend daran angeordneten Schlauchleitungen versehene Kassette 8. Die Kassette 8 ist in eine nicht dargestellte Kammer des Gehäuses 1 einschiebbar und dabei mit einem peristaltisch wirkenden Pumpenrad in Eingriff bringbar. Ein derartiges Irrigations- und Aspirationssystem ist beispielsweise aus der US-A 5,454,783 bekannt.

Fig.2 zeigt die in perspektivischer Ansicht dargestellte ophthalmologische Einrichtung 100 gemäss Fig.1 und man erkennt die vorstehend in Verbindung mit Fig.1 beschriebene Anzeigeeinheit 2, die Druckeinheit 4 sowie das Irrigations- und Aspirationssystem 7 mit der Kassette 8.

Abweichend von der Darstellung gemäss Fig.1 ist in Fig.2 die Beleuchtungseinheit 3 aus einer im Gehäuse 1 vorgesehenen Kammer 3' herausgezogen dargestellt. Die Beleuchtungseinheit 3 umfasst ein Einschubgehäuse 25, welches zur Aufnahme einer mit mehreren Funktionselementen versehenen Vorrichtung 30 (Fig.4) ausgebildet ist. Das Einschubgehäuse 25 ist stirnseitig durch eine Frontabdeckung 28 verschlossen. An der Frontabdeckung 28 sind die beiden mit der Vorrichtung 30 gemäss Fig.4 in Wirkverbindung stehenden Einstellknöpfe 65 und 65' sowie die beiden Anschlussbuchsen 33 und 34 angeordnet. Die Anschlussbuchsen 33,34 sind jeweils mit einem in Fig.2 nicht dargestellten Lichtkanal für die einsteckbaren Beleuchtungssonden 10,10' versehen. Weiterhin erkennt man an der oberen Abdeckplatte 27 des Einschubgehäuses 25 angeordnete und mit dem Innenraum 25' in Verbindung stehende Lüftungsschlitze 27''.

In Fig.3 ist das mit dem Innenraum 25' versehene Einschubgehäuse 25 schematisch und im Schnitt dargestellt und man erkennt die Frontabdeckung 28 mit den beiden im Abstand zueinander angeordneten Einstellknöpfen 65,65', die beiden Seitenwände 26,26', die Rückwand 28' sowie die untere Bodenplatte 27'. Bei dem dargestellten Ausführungsbeispiel sind an der Bodenplatte 27' beispielsweise zwei elektromotorisch angetriebene Ventilatoren 29 und 29' angeordnet. Mittels der Ventilatoren 29,29' wird im wesentlichen die im Innenraum 25' des Einschubgehäuses 25 angeordnete und mit mindestens einer Lichtquelle 40 versehene Vorrichtung 30 (Fig.4) gekühlt. Die Kühlluft wird beispielsweise mittels der beiden in der Bodenplatte 27' angeordneten Ventilatoren 29,29' von unten in den Innenraum 25' eingeblasen und durch die oberen Schlitze 27'' abgeführt.

In dem Innenraum 25' des Einschubgehäuses 25 ist weiterhin mindestens ein Sensor 85 angeordnet mittels welchem die Temperatur in dem Einschubgehäuse 25 überwacht und beim Überschreiten einer eingestellten Betriebstemperatur von etwa 50°C die im Innenraum 25' angeordneten und in nicht dargestellter Weise elektromotorisch angetriebenen Ventilatoren 29 und 29' aktiviert werden. Bei dem bevorzugten Ausführungsbeispiel gemäss Fig.3 sind für die Temperaturüberwachung beispielsweise zwei im Abstand zueinander an der Bodenplatte 27' oder an den Seitenwänden 26,26' angeordnete Sensoren 85 und 85' vorgesehen. Mit dem ersten Sensor 85 kann beispielsweise beim Überschreiten der Betriebstemperatur von etwa 50°C die Leistung der Ventilatoren erhöht und mit dem zweiten Sensor 85' beim Überschreiten der Betriebstemperatur von etwa 70°C die Lichtquelle 40 der Vorrichtung 30 (Fig.4) abgeschaltet werden. Weiterhin kann die Betriebsdauer der Lichtquelle 40 überwacht und diese beim Überschreiten derselben beispielsweise in akustischer oder visueller Form signalisiert werden. Ein visuelles Warnsignal wird vorzugsweise an der Einheit 2 (Fig.1) angezeigt.

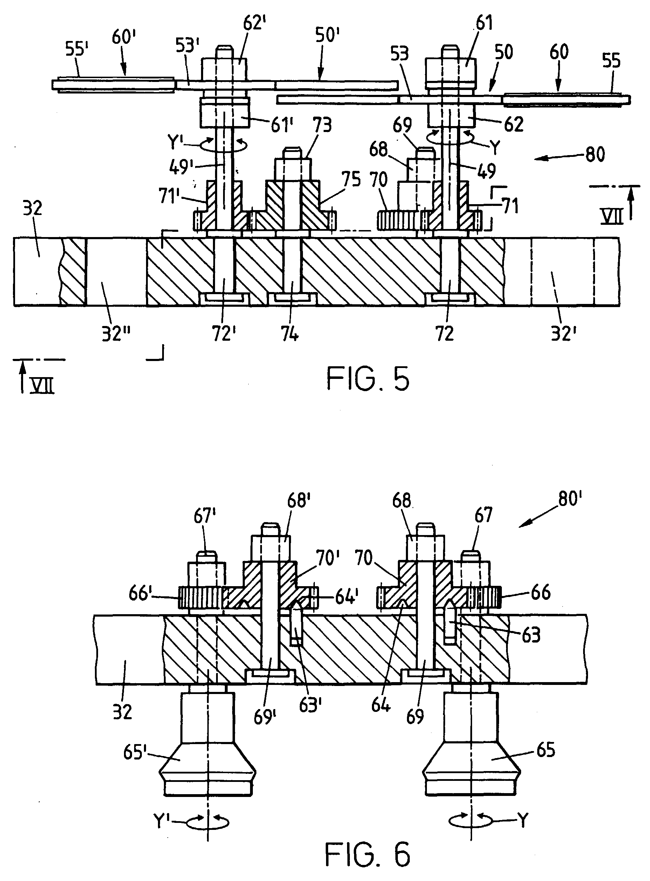

Die beiden stirnseitig an dem Einschubgehäuse 25 angeordneten Einstellknöpfe 65 und 65' (Fig.3) stehen mit einem Getriebe oder dergleichen, beispielsweise mit einem als Stirnradgetriebe 80 und 80' zum Einstellen zwei jeweils um die Längsachse eines Achskörpers in Doppelpfeilrichtung Y und Y' drehbare Blendscheiben 50,50' in Wirkverbindung. Weiterhin besteht die Möglichkeit, dass im Innenraum 25' des Einschubgehäuses 25 ein mit nicht dargestellten Mitteln aktivier- und steuerbarer elektromotorischer Antrieb M angeordnet ist, mittels welchem das Stirnradgetriebe 80 und 80' zum Einstellen der beiden Blendscheiben 50,50' betätigt wird. Die einzelnen mit dem Stirnradgetriebe 80,80' wirkverbundenen Funktionselemente werden später in Verbindung mit den Figuren 5 bis 7 beschrieben.

In Fig.4 ist die mit den einzelnen Funktionselementen versehene Vorrichtung 30 der Beleuchtungseinheit 3 im Schnitt dargestellt und man erkennt zwei im Abstand zueinander angeordnete und für die optischen Elemente ausgebildete Körper 35 und 35'. Im dargestellten Ausführungsbeispiel sind die beiden jeweils als Umlenkelement ausgebildeten Körper 35 und 35' im Profilquerschnitt winkelförmig ausgebildet und mit den beiden horizontalen Schenkeln 36,36' gegeneinander gerichtet im Abstand zueinander angeordnet. Die beiden Körper 35,35' sind an einer Grundplatte 31 angeordnet und mittels nicht dargestellter Schraubverbindungen oder dergleichen befestigt, vorzugsweise auswechselbar befestigt.

Der erste, im Profilquerschnitt winkelförmig ( 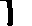 -förmig) ausgebildete Körper 35 hat den mit einem ersten Lichtkanal L1 versehenen ersten Schenkel 36 sowie einen rechtwinklig dazu angeordneten und mit einem zweiten Lichtkanal L2 versehenen zweiten Schenkel 37. In dem zweiten Schenkel 37 ist weiterhin eine etwa spaltförmig ausgebildete Ausnehmung 38 vorgesehen, durch welche der zweite Lichtkanal L2 in zwei nicht bezeichnete Teilstücke unterteilt ist. Der zweite, im Profilquerschnitt winkelförmig ( 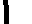 -förmig) ausgebildete Körper 35' ist in bezug auf den ersten Körper 35 spiegelbildlich ausgebildet und umfasst den ersten Schenkel 36' mit darin angeordnetem ersten Lichtkanal L1' sowie einen rechtwinklig dazu angeordneten zweiten Schenkel 37' mit darin angeordnetem zweiten Lichtkanal L2'. Der zweite Schenkel 37' hat ebenfalls eine spaltförmige Ausnehmung 38', durch welche der zweite Lichtkanal L2' in zwei nicht bezeichnete Teilstücke unterteilt ist.

Die beiden etwa quer zu den zweiten Lichtkanälen L2 und L2' orientierten Ausnehmungen 38 und 38' in den Körpern 35 und 35' dienen jeweils zur Aufnahme einer entsprechend gelagerten Blendscheibe 50 beziehungsweise 50'. Die beiden Blendscheiben 50 und 50' sind jeweils durch den in Fig.4 nicht dargestellten und beispielsweise als Stirnradgetriebe ausgebildeten Antrieb 80 und 80', wie in Fig.5 bis Fig.7 dargestellt, um eine theoretische Achse 49 und 49' in Pfeilrichtung Y beziehungsweise Y' drehbar. Die besondere Ausgestaltung und Funktion der beiden im Lichtkanal L2 und L2' angeordneten Blendscheiben 50 und 50' wird später noch im einzelnen beschrieben.

Wie weiterhin in Fig.4 dargestellt ist an dem Ende des zweiten Schenkels 37 und 37' der beiden winkelförmig ausgebildeten Körper 35,35 jeweils die Anschlussbuchse 33 beziehungsweise 34 angeordnet. Die beiden Anschlussbuchsen 33,34 sind an dem einen Ende jeweils mit einem Flansch 33' beziehungsweise 34' sowie mit daran angeformten Absätzen 23 und 24 versehen. Mittels der ringförmigen Absätze 23,24 sind die Anschlussbuchsen 33,34 in dem zugeordneten Lichtkanal L2 beziehungsweise L2' koaxial angeordnet. Die Anschlussbuchsen 33 und 34 sind beispielsweise mit einer nicht dargestellten Schraubverbindung an dem jeweiligen Körper 35,35' lösbar befestigt. Bei dem dargestellten Ausführungsbeispiel sind die Anschlussbuchsen 33,34 an dem anderen Ende jeweils mit einem angeformten und etwa stutzenförmig ausgebildeten Führungsteil 23',24' versehen, an welchen eine Halteplatte 32 gelagert und mittels nicht dargestellter Schraubverbindungen lösbar befestigt ist. Weiterhin erkennt man in Fig.4 in den beiden Anschlussbuchsen 33,34 angeordnete und dieselben in axialer Richtung durchdringende dritte Lichtkanäle L3 und L3', welche mit dem einen Ende mit den zugeordneten Lichtkanälen L2 und L2' der Körper 35,35' korrespondieren. Am anderen Ende sind die dritten Lichtkanäle L3,L3' zum Einstecken der jeweils mit einer entsprechend ausgebildeten Kontakthülse 22,22' versehenen Beleuchtungssonde 10,10' ausgebildet.

Die beiden an der Grundplatte 31 befestigten Körper 35,35' und die daran angeordneten Anschlussbuchsen 33,34 sowie die daran gelagerte Halteplatte 32 bilden zusammen die aus mehreren Funktionselementen als eine Baueinheit ausgebildete Vorrichtung 30, welche nachstehend im einzelnen beschrieben wird.

Bei dem in Fig.4 dargestellten Ausführungsbeispiel umfasst die Vorrichtung 30 mindestens die eine zwischen den beiden Körpern 35,35' auswechselbar angeordnete und schematisch dargestellte Lichtquelle 40, welche ohne die für den elektrischen Anschluss erforderliche Fassung dargestellt ist. Bei der an sich bekannten Lichtquelle 40 handelt es sich beispielsweise um eine elektrische Lichtbogenlampe (micro low-pressure "Longlife" bulb) mit einer Leistung von 20 bis 100 Watt, vorzugsweise jedoch mit einer Leistung von 50 Watt. Die Lichtquelle 40 hat im Vergleich zu den bisher bei derartigen Beleuchtungssystemen eingesetzten Lichtquellen einen relativ hohen Lichtstrom, welcher etwa dem dreifachen einer vergleichbaren 50 W Halogenlampe entspricht. Die eingesetzte Lichtquelle 40 hat zudem eine verhältnismässig geringe Leistungsaufnahme und folglich im Vergleich zu anderen Lichtquellen eine relativ geringe Wärmeentwicklung.

Versuche haben ergeben, dass die Verwendung der Bogenlampe 40 beispielsweise im Vergleich zu der bisher verwendeten Halogenlampe eine etwa 30 mal längere Lebensdauer hat. Das Lichtspektrum der verwendeten Bogenlampe ist gegenüber der Halogenlampe von Vorteil, da die Halogenlampe im infrarot Bereich strahlt und dabei eine relativ hohe Wärmestrahlung erzeugt. Das Lichtspektrum der Bogenlampe ist über den sichtbaren Bereich verhältnismässig gleichmässig und mit einem Maximum von etwa 500 nm im grünen Bereich, in welchem das menschliche Auge die grösste Farbempfindlichkeit aufweist. Zudem wird bei der verwendeten Bogenlampe die Energie von 50 W in wesentlich mehr sichtbares Licht umgewandelt, während eine Halogenlampe wesentlich mehr Wärme erzeugt und abstrahlt. Die Bogenlampe wird nachstehend generell als Lichtquelle 40 bezeichnet.

Zu beiden Seiten der Lichtquelle 40 ist jeweils eine erste optische Linse 47,47' angeordnet, welche eingangsseitig in dem ersten Lichtkanal L1 beziehungsweise L1' der beiden im Abstand zueinander angeordneten Schenkel 36 und 36' der Körper 35,35' auswechselbar angeordnet sind. Die beiden ersten optischen Linsen 47,47' sind vorzugsweise möglichst nahe im Bereich der Lichtquelle 40 angeordnet. Im dargestellten Ausführungsbeispiel sind die beiden ersten Linsen 47,47' auf der der Lichtquelle 40 abgewandten Seite beispielsweise mit einer konvex nach aussen gewölbten oder mit einer sphärisch beziehungsweise parabolisch ausgebildeten Fläche (nicht bezeichnet) versehen. Auf der gegenüberliegenden Seite sind die beiden Linsen 47,47' jeweils durch eine mit einer Öffnung 41,41' versehenen Scheibe 42 und 42' oder dergleichen an dem Körper 35 beziehungsweise 35' gehalten sowie durch eine nicht dargestellte Schraubverbindung auswechselbar am Körper 35,35' befestigt. Die von der Lichtquelle 40 abgegebenen beziehungsweise reflektierten Lichtstrahlen (nicht bezeichnet) durchdringen die Öffnung 41,41' der Scheiben 42,42' sowie die ersten Linsen 47,47' und werden durch den ersten Lichtkanal L1,L1' auf ein entsprechend zugeordnetes und die abgestrahlte Wärme durchlassendes beziehungsweise auskoppelndes Bauteil 43,43' gerichtet. Das einzelne die Wärme auskoppelnde Bauteil wird nachstehend als Umlenkspiegel 43,43' bezeichnet, wobei die Wärme den jeweiligen Umlenkspiegel 43 oder 43' passiert während das sichtbare Licht gespiegelt (umgelenkt) wird.

Die beiden Umlenkspiegel 43,43' sind beispielsweise aus hitzebeständigem Material, vorzugsweise aus hitzebeständigem Glas hergestellt. Zur Erreichung einer Filterwirkung sind die beiden Umlenkspiegel 43,43' auf der einen Seite entsprechend (Reflextion R 97% bei 425 bis 600 nm; Durchlässigkeit T 50% bei 685 nm; Durchlässigkeit T 80% bei 750 bis 2500 nm) bedampft (nm = Nanometer als Masseinheit der Wellenlänge).

Die beiden dem ersten Lichtkanal L1,L1' zugeordneten Umlenkspiegel 43 und 43' sind an einer abgeschrägten Aussenfläche 39,39' des jeweiligen Körpers 35 und 35' angeordnet. Der einzelne Umlenkspiegel 43,43' ist in bezug auf den zugeordneten ersten Lichtkanal L1 und L1' unter einem Winkel von 45° dazu angeordnet. Im dargestellten Ausführungsbeispiel sind die beiden Umlenkspiegel 43,43' jeweils aussenseitig an der unter einem Winkel von 45° abgeschrägt ausgebildeten Fläche 39,39' des einzelnen Körpers 35 beziehungsweise 35' angeordnet. Die Umlenkspiegel 43,43' sind beispielsweise durch eine nicht dargestellte Schraubverbindung auswechselbar an dem Körper 35,35' befestigt.

Bei einem weiteren Ausführungsbeispiel kann das als Umlenkspiegel 43,43' ausgebildete Bauteil in nicht näher dargestellter Weise mit einer Filtereinheit versehen werden, welche beispielsweise einen Hitze- sowie einen UV-Filter umfasst. Mit der zusätzlichen Filtereinheit wird erreicht, dass von dem Hitzefilter die Wärmestrahlung nach aussen abgegeben und von dem UV-Filter die restliche UV-Strahlung absorbiert wird.

In dem abgewinkelt, vorzugsweise rechtwinklig zu dem ersten Lichtkanal L1 beziehungsweise L1' orientierten zweiten Lichtkanal L2 beziehungsweise L2' ist unmittelbar im Bereich des ersten Lichtkanals L1 und L1' jeweils eine Filtereinheit F beziehungsweise F' angeordnet. Die beiden Filtereinheiten F,F' sind durch geeignete Mittel in den zweiten Lichtkanälen L2,L2' der zweiten Schenkel 37,37' gehalten. Im dargestellten Ausführungsbeispiel umfasst die einzelne Filtereinheit F und F' einen Hitzefilter 45 und 45' sowie einen nachgeordneten UV-Filter 46 und 46'. Die beiden Filtereinheiten F und F' sind im dargestellten Ausführungsbeispiel jeweils durch einen eingesetzten Sicherungsring, beispielsweise durch einen Sprengring 44 und 44' in dem zweiten Lichtkanal L2,L2' auswechselbar angeordnet.

Die beiden Hitzefilter 45 und 45' sind beispielsweise aus hitzebeständigem Glas hergestellt, mittels welcher die von den beiden Umlenkspiegeln 43 und 43' kommende Wärme blockiert wird. Von den beiden zugeordneten UV-Filtern wird die auf das menschliche Auge toxisch wirkende UV-Strahlung bis 440 nm (50%) absorbiert. Die beiden Hitzefilter 45 und 45' sind vorzugsweise wie nachstehend aufgeführt bedampft: Durchlässigkeit **T** < 1% bis 380 nm; **T** 50% bei 400 nm; **T** > 80% bei 425 - 680 nm; **T** = 50% bei 730 nm; **T** < 3% bei 800 - 1150 nm; **T** < 10% bei 1150 - 2500 nm.

Weiterhin erkennt man in Fig.4 die jeweils in der Ausnehmung 38 und 38' der zweiten Lichtkanäle L2 und L2' angeordneten Blendscheiben 50,50' sowie die nachgeschalteten zweiten optischen Linsen 48 und 48', welche jeweils am Ende des zweiten Lichtkanals L2,L2' in dem zweiten Schenkel 37,37' des Körpers 35,35' angeordnet sind. Die zweiten Linsen 48 und 48' sind auf der der Blendscheibe 50 beziehungsweise 50' zugewandten Seite jeweils mit einer sphärische Fläche (nicht bezeichnet) versehen und werden auf der gegenüberliegenden Seite jeweils durch einen an der Anschlussbuchse 33 und 34 angeformten Absatz 23 und 24 in dem Körper 35,35' gehalten. Die mit den dritten Lichtkanälen L3,L3' versehenen Anschlussbuchsen 33 und 34 sind jeweils mit einem angeformten Flansch 33' und 34' an dem Körper 35,35' angeordnet und mit nicht dargestellten Befestigungsmitteln, vorzugsweise durch eine Schraubverbindung lösbar befestigt.

Bei der vorstehend beschriebenen Vorrichtung 30 wird das von der Lichtquelle 40 abgestrahlte Licht von der zugeordneten ersten optischen Linse 47 oder 47' aufgenommen und parallel durch den ersten Lichtkanal L1,L1' auf den Umlenkspiegel 43,43' geleitet. Hierbei durchdringt die Strahlung von etwa 750nm bis 2500nm (Wärme oder Hitze) den Umlenkspiegel 43,43' und wird ausgekoppelt, während die von dem Umlenkspiegel 43,43' reflektierte sichtbare Strahlung von etwa 425nm bis 685nm in den zweiten Lichtkanal L2,L2' umgelenkt wird. In dem zweiten Lichtkanal L2,L2' trifft die Strahlung zuerst auf den zugeordneten Hitzefilter 45,45', von welchem die restliche Wärmestrahlung von beispielsweise über 730nm sowie die UV-Strahlung unter 400nm auf den Umlenkspiegel 43,43' reflektiert und von diesem nach aussen abgegeben oder absorbiert wird. Die noch vorhandene Strahlung von unter etwa 440nm wird von dem nachgeschalteten UV-Filter 46 und 46' absorbiert. Die verbleibenden Lichtstrahlen werden über die zwischengeschaltete und mit einer entsprechenden Abblendfläche in den zweiten Lichtkanal L2,L2' einschwenkbare Blendscheibe 50 und 50' durchdringend auf die zweite optische Linse 48 oder 48' geleitet und von dieser im wesentlichen auf die Beleuchtungssonde 10 oder 10' fokussiert. Bei einem bevorzugten Ausführungsbeispiel können die Lichtstrahlen auch einen jeweils in den zweiten Lichtkanal L2,L2' einschwenkbaren Farbfilter 60 (Grünfilter) oder einen Fluoreszensfilter 60' (Fig.5) durchdringend auf die zweite optische Linse 48 oder 48' geleitet werden und von diesen auf die zugeordnete Beleuchtungssonde 10 oder 10' fokussiert werden. Der Farbfilter 60 ist vorzugsweise an der einen Blendscheibe 50 und der Fluoreszensfilter 60' an der anderen Blendscheibe 50' angeordnet.

Bei der in Fig.4 dargestellten Vorrichtung 30 wird der Lichtstrom von der ersten optischen Linse 47;47' durch einen ersten Lichtkanal L1;L1' auf den zugeordneten und die Wärme mindestens teilweise auskoppelnden Umlenkspiegel 43;43' geleitet und von diesem die sichtbare Strahlung in den abgewinkelt zu dem ersten Lichtkanal L1;L1' orientierten zweiten Lichtkanal L2;L2' umgelenkt. In dem zweiten Lichtkanal L2;L2' durchdringt die sichtbare Strahlung die zur wahlweisen Regulierung der Lichtintensität ausgebildete Blendscheibe 50;50' und wird danach auf die zweite optische Linse 48;48' geleitet und von dieser auf die Beleuchtungssonde 10;10' fokussiert.

Bei einer nicht dargestellte Variante der Vorrichtung 30 besteht jedoch auch die Möglichkeit, dass die jeweils in den abgewinkelt zu dem ersten Lichtkanal L1 oder L1' orientierten zweiten Lichtkanal L2 oder L2' umgelenkte sichtbare Strahlung in einer ersten Phase auf die zweite optische Linse 48 oder 48' und von dieser zur wahlweisen Regulierung der Lichtintensität die nachgeschaltete Blendscheibe 50 oder 50' durchdringend auf die Beleuchtungssonde 10 oder 10' fokussiert wird.

Bei den vorstehend in Verbindung mit Fig.4 beschriebenen und als Ausführungsbeispiel schematisch dargestellten sowie in den Lichtkanälen L1,L2 beziehungweise L1',L2' der Körper 35,35' angeordneten optischen Linsen handelt es sich beispielsweise um plan-konvex ausgebildete optische Linsen 47,48 und 47',48'. Bei den optischen Linsen 47,48 beziehungsweise 47',48' ist das nicht bezeichnete und jeweils in den zugeordneten Lichtkanal L1,L2 beziehungsweise L1',L2' orientierte Linsenteil mit konvex gewölbter oder mit einer sphärischen beziehungsweise parabolischen Formgebung versehen. Bei einer nicht dargestellten Variante besteht jedoch auch die Möglichkeit, dass die der Lichtquelle 40 zugewandte Linsenseite (nicht bezeichnet) der beiden ersten optischen Linsen 47 und 47' jeweils geringfügig konvex gewölbt ausgebildet ist.

Bei einer weiteren nicht dargestellten Variante besteht zudem die Möglichkeit, dass die in den abgewinkelt zueinander angeordneten Lichtkanälen L1,L2 beziehungweise L1',L2' eingesetzten optischen Linsen 47,48 beziehungsweise 47',48' entweder bi-konvex oder aber konkav-konvex ausgebildet sind.

In Fig.5 ist der eine beispielsweise als Stirnradgetriebe ausgebildete Antrieb 80 für die beiden um die Achse 49 und 49' in Pfeilrichtung Y beziehungsweise Y' drehbaren Blendscheiben 50,50' und der daran angeordneten Farbfilter 60 (Grünfilter) beziehungsweise Fluoreszensfilter 60' dargestellt. Der Antrieb 80 umfasst zwei in parallelem Abstand zueinander angeordnete und mit dem einen Ende in der Halteplatte 32 gelagerte erste Achskörper 72 und 72'. An dem der Halteplatte 32 abgewandten vorderen Ende der beiden Achskörper 72,72' ist jeweils die Blendscheibe 50,50' angeordnet und gelagert. Die beiden Blendscheiben 50,50' sind je durch eine auf den Achskörper 72,72' aufgeschraubte Mutter 61,61' sowie durch einen Stellring 62,62' an dem jeweiligen Achskörper 72,72' angeordnet und gegen axiales Verschieben gesichert. Die beiden Blendscheiben 50,50' sind weiterhin mittels der Elemente 61,61' und 62,62' mit dem Achskörper 72,72' um dessen Längsachse 49,49' drehfest wirkverbunden. Weiterhin erkennt man in Fig.5 die in der Halteplatte 32 im Abstand zueinander angeordneten Öffnungen 32',32'' für die beiden mit dem Führungsteil 23',24' darin gelagerten Anschlussbuchsen 33,34 (Fig.4).

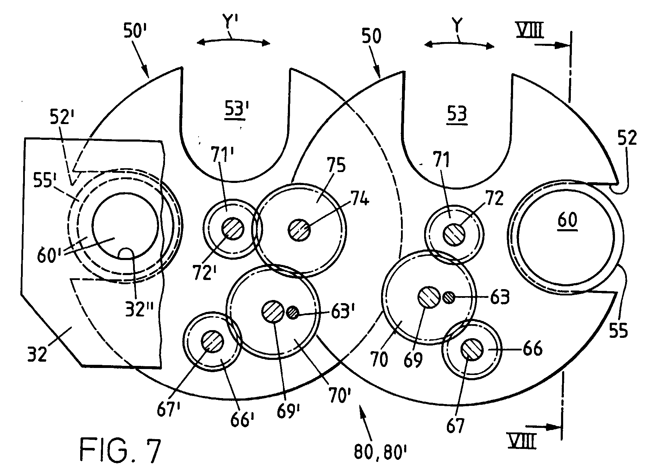

In axialem Abstand zu der ersten Blendscheibe 50 ist an dem ersten Achskörper 72 ein damit drehtest wirkverbundenes Zahnrad 71 gelagert, welches über ein zugeordnetes erstes Rasterrad 70 mit der Aussenverzahnung eines ersten Antriebsrads 66 (Fig.6,7) in Eingriff steht. In axialem Abstand zu der zweiten Blendscheibe 50' ist an dem zweiten Achskörper 72' ein damit drehfest wirkverbundenes Zahnrad 71' gelagert, welches über ein zugeordnetes Stirnrad 75 und einem damit wirkverbundenen zweiten Rasterrad 70' mit der Aussenverzahnung eines zweiten Antriebsrads 66' (Fig.6,7) in Eingriff steht. Das Stirnrad 75 ist ebenfalls an einem in der Halteplatte 32 gelagerten Achskörper 74 angeordnet und durch einen Stellring 73 gegen axiales Verschieben an dem Achskörper 74 gesichert.

Fig.6 zeigt ein Teilstück des zweiten beispielsweise als Stirnradgetriebe ausgebildeten Antriebs 80' und man erkennt die Halteplatte 32 sowie die beiden daran im Abstand zueinander angeordneten Einstellknöpfe 65 und 65'. Die analog der Blendscheiben 50,50' in Pfeilrichtung Y beziehungsweise Y' drehbaren Einstellknöpfe 65,65' sind in nicht dargestellter Weise mit einer Antriebswelle 67,67' wirkverbunden. An dem anderen Ende der Antriebswelle 67,67' ist je ein drehfest damit wirkverbundenes und mit einer Aussenverzahnung versehenes Antriebsrad 66,66' angeordnet. Das einzelne Antriebsrad 66, 66' steht mit der Aussenverzahnung des zugeordneten und jeweils an einem Achskörper 69,69' gelagerten Rasterrades 70,70' in Wirkverbindung. Das einzelne Rasterrad 70,70' ist auf der der Halteplatte 32 zugewandten Seite mit mehreren in Umfangsrichtung verteilt zueinander angeordneten Kerben 64,64' versehen in welche, wie in Fig.6 schematisch dargestellt, ein Rasterzapfen 63,63' eingreift. Bei der Drehbewegung des Rasterrades 70,70' wird der Rasterzapfen 63,63' gegen die Rückstellkraft eines nicht dargestellten Federelements aus der jeweiligen Kerbe 64,64' herausgedrückt um anschliessend in die nächste Kerbe einzurasten. Die beiden Rasterzapfen 63,63' sind in nicht näher dargestellter Weise in der Halteplatte 32 angeordnet und gesichert. Weiterhin erkennt man in Fig.6 das durch einen Stellring 68,68' an dem Achskörper 69,69' gegen axiales Verschieben gesicherte Rasterrad 70,70'.

Fig.7 zeigt einen Schnitt gemäss der in Fig.5 eingezeichneten Linie VII-VII und man erkennt ein Teilstück der Halteplatte 32 mit der einen Öffnung 32'' sowie die beiden in Ansicht dargestellten Blendscheiben 50 und 50' und die einzelnen Funktionselemente des Antriebs 80 und 80'. Die Blendscheiben 50,50' sind jeweils mit zwei um einen Winkel von 90° versetzt zueinander angeordneten Ausnehmungen 52,53 beziehungsweise 52',53' versehen. In den beiden ersten Ausnehmungen 52 und 52' ist jeweils der Farbfilter 60 beziehungsweise der Fluoreszensfilter 60' angeordnet, welche jeweils mit dem entsprechend ausgebildeten Haltering 55,55' an der Blendscheibe 50,50' auswechselbar gehalten sind. Weiterhin erkennt man den zur Lagerung der Blendscheibe 50,50' ausgebildeten Achskörper 72,72' sowie das daran angeordnete Zahnrad 71,71'. Das eine Zahnrad 71 steht mit dem am Achskörper 69 gelagerten ersten Rasterrad 70 und dieses mit dem an der ersten Antriebswelle 67 gelagerten Antriebsrad 66 in Eingriff. Das andere Zahnrad 71' steht über das zugeordnete Stirnrad 75 mit dem am Achskörper 69' gelagerten zweiten Rasterrad 70' und dieses mit dem an der zweiten Antriebswelle 67' gelagerten zweiten Antriebsrad 66' in Eingriff.

Die beiden Blendscheiben 50,50' sind mittels dem als Stirnradgetriebe ausgebildeten und entsprechend zugeordneten Antrieb 80,80' um die Längsachsen 49,49' (Fig.5) der beiden Achskörper 72,72' in Doppelpfeilrichtung Y,Y' drehbar. Die Betätigung der Antriebe 80,80' kann dabei mittels der beiden Einstellknöpfe 65,65' oder mittels des entsprechend ansteuerbaren elektromotorischen Antriebs M (Fig.3) getrennt oder miteinander erfolgen.

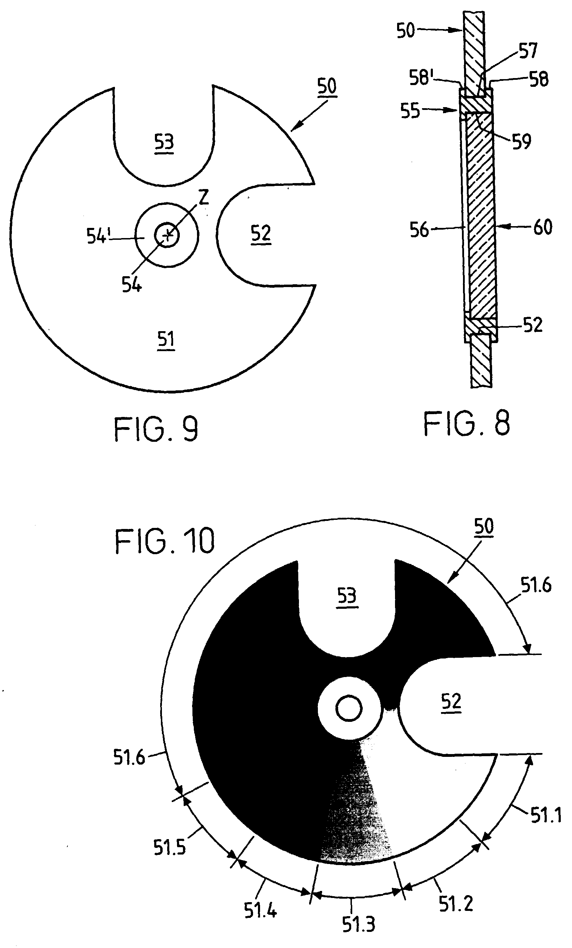

Fig.8 zeigt die eine im Schnitt sowie in grösserem Massstab dargestellte Blendscheibe 50 mit dem daran angeordneten Haltering 55 für den Farbfilter 60. Der Haltering 55 ist beispielsweise mit einer kreisförmigen Öffnung 56 sowie mit einer kreisförmigen Ausnehmung 59 versehen, wobei die Ausnehmung 59 zur Aufnahme des Farbfilters 60 ausgebildet ist. Der Farbfilter 60 ist mit nicht dargestellten Befestigungsmitteln auswechselbar in der Ausnehmung 59 des Halterings 55 angeordnet. Am äusseren Umfang des Halterings 55 ist eine durch seitliche Stege 58,58' begrenzte Ringnut 57 angeordnet. Die Ringnut 57 mit den beiden Stegen 58,58' ist derart ausgebildet, dass in montiertem Zustand der Haltering 55 in der Ausnehmung 52 der Blendscheibe 50 gehalten ist. Der Haltering 55' für den Fluoreszensfilter 60' (Fig.5,7) ist analog dem Haltering 55 für den Farbfilter 60 ausgebildet.

In Fig.9 ist die erste Blendscheibe 50 für die Vorrichtung 30 (Fig.4) als Rohling sowie in Draufsicht dargestellt. Im Zentrum Z der Blendscheibe 50 ist eine Durchgangsbohrung 54 angeordnet, welche an der Oberfläche 51 der Blendscheibe 50 von einer kreisringförmigen Anlagefläche 54' umgeben ist. Weiterhin ist die Blendscheibe 50 mit den beiden um einen Winkel von 90° versetzt zueinander angeordneten Ausnehmungen 52 und 53 versehen. Die erste Ausnehmung 52 ist wie vorstehend in Verbindung mit Fig.8 beschrieben zur Aufnahme des mit dem Farbfilter 60 versehenen Halterings 55 ausgebildet.

Fig.10 zeigt die in Draufsicht dargestellte erste Blendscheibe 50 mit den beiden Ausnehmungen 52 und 53. Ausgehend von der ersten Ausnehmung 52 ist die Blendscheibe 50 im Uhrzeigersinn gesehen mit mehreren Abblendflächen versehen, mittels welcher in Abhängigkeit der Drehrichtung die Lichtmenge, welche die Blendscheibe 50 durchdringt entsprechend regelbar ist. Die einzelnen vorzugsweise kontinuierlich ineinander übergehenden Abblendflächen sind durch geeignete Mittel, beispielsweise nach dem an sich bekannten Siebdruckverfahren auf die Oberfläche des Blendscheiben-Rohlings 50 (Fig.9) aufgebracht.

Bei dem dargestellten Ausführungsbeispiel sind die einzelnen Abblendflächen 51.1 bis 51.6 ausgehend von dem Zentrum Z (Fig.9) etwa als kreissektorförmig nach aussen erweiternde Flächen ausgebildet. Ausgehend von der ersten Abblendfläche 51.1 sind die einzelnen daran anschliessenden Abblendflächen 51.2 bis 51.6 mit zunehmender Punktdichte, vorzugsweise mit kontinuierlich zunehmender Punktdichte versehen. Bei dem in Fig.10 dargestellten Ausführungsbeispiel ist die Punktdichte der Abblendfläche 51.6 so gewählt, dass die durch die zweite Ausnehmung 53 unterteilte Fläche der Abblendfläche 51.6 weitgehend lichtundurchlässig (schwarz) ist.

Bei der in Fig.10 als Ausführungsbeispiel dargestellten ersten Blendscheibe 50 ist eine gleichmässig ineinanderübergehende Transparenz von 8 Flächen vorgesehen, wobei die in der Blendscheibe 50 angeordnete Ausnehmung 53 eine maximale Lichtdurchlässigkeit (Transparenz) von 100% gewährleistet. Die zweite Ausnehmung 52 dient zur Aufnahme des mit dem Farbfilter 60, beispielsweise mit einem Grünfilter 60 versehenen Halterings 55.

Die zweite Blendscheibe 50' ist spiegelbildlich und analog der vorstehend beschriebenen ersten Blendscheibe 50 ausgebildet und mit den entsprechend angeordneten und ausgebildeten Abblendflächen 51.1 bis 51.6 versehen. Abweichend von der ersten Blendscheibe 50 ist bei der zweiten Blendscheibe 50' in der zweiten Ausnehmung 52' der andere Haltering 55' mit dem Fluoreszenzfilter 60' angeordnet (Fig.7).

Die beiden Blendscheiben 50 und 50' sind vorzugsweise aus hitzebeständigem Glas hergestellt und beispielsweise mit hitzebeständiger oder keramischer Farbe bedruckt oder metallisch beschichtet. Mit dem an der ersten Blendscheibe 50 angeordneten Farbfilter 60 (Grünfilter) wird in vorteilhafter Weise das Erkennen relativ dünner Membranen im menschlichen Auge ermöglicht. Mit dem an der zweiten Blendscheibe 50' angeordneten Fluoreszenzfilter 60' wird bei gleichzeitiger Initierung einer als Kontrastmittel wirkenden Substanz eine spezielle Methode für die visuelle Erkennung der Blutadern im Auge erreicht. Das initierte Kontrastmittel reagiert dabei mit dem Licht aus dem Fluoreszenzfilter 60' derart, dass das Licht in einem anderen Wellenlängenbereich leuchtet und dabei mittels eines nicht dargestellten und beispielsweise an einem Mikroskop angeordneten Betrachtungsfilters erkennbar wird. Der Betrachtungsfilter sperrt dabei das Licht aus dem Fluoreszenzfilter 60' und lässt das Licht des Kontrastmittels passieren.

An dieser Stelle wird darauf hingewiesen, dass die in Fig.2 aus dem Gehäuse 1 der ophthalmologischen Einrichtung 100 herausgezogene und mit den angeschlossenen Beleuchtungssonden 10 und 10' versehene Beleuchtungseinheit 3 mit den darin angeordneten und vorstehend anhand der Figuren 3 bis 10 beschriebenen Funktionselementen auch als eine einzelne getrennte Baueinheit für die ophthalmologische Aussen- und/oder Innenbeleuchtung des Auges 20 (Fig.1) verwendet werden kann.

Die verfahrenstechnischen Merkmale sowie die Vorrichtung mit den entsprechend angeordneten optischen Elementen zur Durchführung des Verfahrens sind nicht auf das vorstehend beschriebene Ausführungsbeispiel, insbesondere nicht auf die in Fig.4 dargestellte Vorrichtung 30 beschränkt. Weitere zweckmässige Ausgestaltungen und Anordnungen der einzelnen Funktionselemente sind ebenfalls möglich, ohne dabei den Grundgedanken eines auf eine Beleuchtungssonde zu übertragenden gleichbleibenden Lichtkegeldurchmessers mit möglichst geringer Wärmeentwicklung erreicht wird.

## Patentansprüche

1. Verfahren zum Fokussieren eines von einer Lichtquelle abgestrahlten Lichtstromes, welcher über mindestens eine mit einem Lichtkanal in Verbindung stehende optische Linse auf einen Lichtleiter, insbesondere auf einen als ophthalmoskopische Beleuchtungssonde ausgebildeten Lichtleiter fokussiert wird, **dadurch gekennzeichnet**, dass der Lichtstrom von einer ersten optischen Linse (47) durch einen ersten Lichtkanal (L1) auf ein zugeordnetes und die Wärme mindestens teilweise auskoppelndes Bauteil (43) geleitet und von diesem die sichtbare Strahlung in einen abgewinkelt zu dem ersten Lichtkanal (L1) orientierten zweiten Lichtkanal (L2) umgelenkt wird, wobei die sichtbare Strahlung in dem zweiten Lichtkanal (L2) entweder zuerst eine zur wahlweisen Regulierung der Lichtintensität ausgebildete Blendscheibe (50) durchdringend auf eine zweite optische Linse (48) geleitet und von dieser auf die Beleuchtungssonde (10) fokussiert wird beziehungsweise zuerst auf die zweite optische Linse (48) und von dieser die in Richtung der sichtbaren Strahlung nachgeschaltete Blendscheibe (50) durchdringend auf die Beleuchtungssonde (10) fokussiert wird.

2. Verfahren nach Anspruch 1, **dadurch gekennzeichnet**, dass der Lichtstrom von beidseitig der Lichtquelle (40) angeordneten ersten optischen Linsen (47;47') durch den ersten Lichtkanal (L1;L1') auf das jeweils die Wärme auskoppelnde Bauteil (43;43') geleitet und von diesem die sichtbare Strahlung in den zweiten Lichtkanal (L2;L2') umgelenkt und dabei zur Regelung der Lichtintensität die mit mehreren Abblendflächen (51.1 bis 51.6) versehene und in bezug auf den zweiten Lichtkanal (L2;L2') einstellbare Blendscheibe (50;50') durchdringend auf die zweite optische Linse (48;48') geleitet und von dieser auf die Beleuchtungssonde (10;10') fokussiert wird.

3. Verfahren nach Anspruch 1, **dadurch gekennzeichnet**, dass der Lichtstrom von beidseitig der Lichtquelle (40) angeordneten ersten optischen Linsen (47;47') durch den ersten Lichtkanal (L1;L1') auf das jeweils die Wärme auskoppelnde Bauteil (43;43') geleitet und von diesem die sichtbare Strahlung in den zweiten Lichtkanal (L2;L2') umgelenkt und dabei zuerst auf die zweite optische Linse (48;48') und anschliessend zur Regelung der Lichtintensität die mit mehreren Abblendflächen (51.1 bis 51.6) versehene und in bezug auf den zweiten Lichtkanal (L2;L2') einstellbare Blendscheibe (50;50') durchdringend auf die Beleuchtungssonde (10;10') fokussiert wird.

4. Verfahren nach den Ansprüchen 2 und 3, **dadurch gekennzeichnet**, dass die jeweils von dem Bauteil (43,43') in den zugeordneten zweiten Lichtkanal (L2;L2') umgelenkte sichtbare Strahlung hinsichtlich der Lichtintensität wahlweise durch eine der Abblendflächen (51.1 bis 51.6) der Blendscheibe (50; 50') oder durch einen vorzugsweise an der einen Blendscheibe (50) angeordneten Farbfilter (60) beziehungsweise durch einen vorzugsweise an der anderen Blendscheibe (50') angeordneten Fluoreszenzfilter (60') reguliert wird.

5. Verfahren nach einem der Ansprüche 2 bis 4, **dadurch gekennzeichnet**, dass die jeweils von dem Bauteil (43;43') in den zugeordneten zweiten Lichtkanal (L2;L2') umgelenkte sichtbare Strahlung zuerst eine mit einem Hitzefilter (45; 45') sowie mit einem UV-Filter (46;46') versehene Filtereinheit (F;F') durchdringt und anschliessend hinsichtlich der Lichtintensität durch eine der Abblendflächen (51.1 bis 51.6) der Blendscheibe (50;50') oder durch den vorzugsweise an der einen Blendscheibe (50) angeordneten Farbfilter (60) beziehungsweise durch den vorzugsweise an der anderen Blendscheibe (50') angeordneten Fluoreszenzfilter (60') reguliert wird.

6. Verfahren nach einem der Ansprüche 1 bis 5, **dadurch gekennzeichnet**, dass der abgestrahlte Lichtstrom von der mindestens auf der der Lichtquelle (40) abgewandten Seite mit konvex gewölbter Fläche versehenen ersten optischen Linse (47;47') und die umgelenkte sichtbare Strahlung von der mindestens auf der dem Bauteil (43;43') beziehungsweise auf der der Blendscheibe (50;50') zugewandten Seite mit konvex gewölbter Fläche versehenen zweiten optischen Linse (48;48') auf die Beleuchtungssonde (10;10') fokussiert wird.

7. Vorrichtung zum Fokussieren des von einer Lichtquelle abgestrahlten Lichtstromes, welcher über mindestens eine mit einem Lichtkanal in Verbindung stehende optische Linse auf einen Lichtleiter, insbesondere auf einen als ophthalmoskopische Beleuchtungssonde ausgebildeten Lichtleiter fokussierbar ist, **gekennzeichnet durch** mindestens einen der Lichtquelle (40) zugeordneten Körper (35) mit in Richtung des abgestrahlten Lichtstromes orientiertem ersten Lichtkanal (L1) und abgewinkelt dazu angeordnetem zweiten Lichtkanal (L2), und dass eingangsseitig des ersten Lichtkanals (L1) eine erste optische Linse (47) und ausgangsseitig ein die sichtbare Strahlung in den zweiten Lichtkanal (L2) umlenkendes Bauteil (43) angeordnet ist, wobei in dem zweiten Lichtkanal (L2) in Richtung der umgelenkten Strahlung gesehen entweder zuerst eine zur Regulierung der Lichtintensität ausgebildete sowie in bezug auf den zweiten Lichtkanal (L2) einstellbare Blendscheibe (50) und dieser nachgeordnet eine die umgelenkte Strahlung fokussierende zweite optische Linse (48) oder umgekehrt in Richtung der umgelenkten Strahlung gesehen zuerst die zweite Linse (48) und dieser nachgeordnet die Blendscheibe (50) angeordnet ist.

8. Vorrichtung nach Anspruch 7, **dadurch gekennzeichnet**, dass zu beiden Seiten der Lichtquelle (40) sowie im Abstand dazu ein Körper (35;35') angeordnet ist, welcher jeweils den in Richtung des abgestrahlten Lichtstromes orientierten ersten Lichtkanal (L1;L1'), die eingangsseitig darin angeordnete erste optische Linse (47;47'), das ausgangsseitig angeordnete Bauteil (43;43') für die Umlenkung der sichtbaren Strahlung und den zweiten Lichtkanal (L2;L2') mit der Blendscheibe (50;50') sowie die ausgangsseitig angeordnete zweite optische Linse (48;48') umfasst.

9. Vorrichtung nach einem der Ansprüche 7 und 8, **dadurch gekennzeichnet**, dass die in dem ersten Lichtkanal (L1;L1') angeordnete erste optische Linse (47;47') auf der der Lichtquelle (40) abgewandten Seite und die in dem zweiten Lichtkanal (L2;L2') angeordnete zweite optische Linse (48;48') auf der dem Bauteil (43;43') zugewandten Seite jeweils konkav gewölbt ausgebildet sind.

10. Vorrichtung nach einem der Ansprüche 7 und 8, **dadurch gekennzeichnet**, dass die erste sowie die zweite optische Linse (47,48;47'48') plan-gewölbt konvex ausgebildet sind, wobei die erste Linse (47;47') auf der der Lichtquelle (40) abgewandten Seite sowie die zweite Linse (48;48') auf der dem Bauteil (43;43') zugewandten Seite jeweils gewölbt konvex ausgebildet sind.

11. Vorrichtung nach einem der Ansprüche 7 und 8, **dadurch gekennzeichnet,** dass die erste sowie die zweite optische Linse (47,48;47'48') plan-gewölbt ausgebildet sind, wobei die erste Linse (47;47') auf der der Lichtquelle (40) abgewandten Seite und die zweite Linse (48;48') auf der dem Bauteil (43;43') zugewandten Seite mit einer sphärischen oder asphärischen Formgebung versehen sind.

12. Vorrichtung nach einem der Ansprüche 7 und 8, **dadurch gekennzeichnet,** dass der einzelne Körper (35;35') als ein mit den beiden abgewinkelt zueinander orientierten Lichtkanälen (L1,L2;L1',L2') versehener und im Profilquerschnitt etwa winkelförmig ausgebildetes Umlenkelement ausgebildet ist, wobei die jeweils mit dem ersten Lichtkanal (L1;L1') versehenen ersten Schenkel (36;36') im Abstand zu der Lichtquelle (40) und die jeweils mit dem zweiten Lichtkanal (L2;L2') versehenen zweiten Schenkel (37;37') im Abstand zueinander, beispielsweise in parallelem Abstand zueinander angeordnet sind.

13. Vorrichtung nach einem der Ansprüche 7 und 8, **dadurch gekennzeichnet,** dass in dem zweiten Lichtkanal (L2;L2') im Bereich des Bauteils (43;43') je eine Filtereinheit (F;F') angeordnet ist, welche jeweils einen Hitzefilter (45;45') sowie einen UV-Filter (46;46') aufweist.

14. Vorrichtung nach einem der Ansprüche 7 bis 13, **dadurch gekennzeichnet,** dass das ausgangsseitig des ersten Lichtkanals (L1;L1') an dem Körper (35;35') angeordnete Bauteil (43; 43') jeweils als ein die Wärme auskoppelnder und die sichtbare Strahlung umlenkender Spiegel ausgebildet ist.

15. Vorrichtung nach den Ansprüchen 7 und 8, **dadurch gekennzeichnet,** dass die beiden in bezug auf den jeweiligen Lichtkanal (L2;L2') einstellbaren Blendscheiben (50;50') für die Regulierung der Lichtintensität mit mehreren Abblendflächen (51.1 bis 51.6) sowie je mit zwei in Umfangsrichtung versetzt zueinander angeordneten Ausnehmungen (52,53;52',53') versehen sind, von welchen mindestens eine der Ausnehmung (52;52') zur auswechselbaren Aufnahme eines in den zweiten Lichtkanal (L2;L2') einschwenkbaren Filters (60;60') ausgebildet ist.

16. Vorrichtung nach Anspruch 15, **dadurch gekennzeichnet,** dass in der Ausnehmung (52) der ersten Blendscheibe (50) ein Farbfilter (60) und in der Ausnehmung (52') der zweiten Blendscheibe (50') ein Fluoreszenzfilter (60') angeordnet, vorzugsweise auswechselbar angeordnet ist.

17. Vorrichtung nach einem der Ansprüche 7 und 15, **dadurch gekennzeichnet,** dass die an der Blendscheibe (50;50') angeordneten und etwa kreissektorförmig ausgebildeten Abblendflächen (51.1 bis 51.6) ausgehend von der ersten Abblendfläche (51.1) in Umfangsrichtung bis zu der letzten Abblendfläche (51.6) mit kontinuierlich zunehmender Punktdichte versehen sind, wobei die letzte Abblendfläche (51.6) zur Erreichung einer weitgehenden Lichtundurchlässigkeit schwarz ausgebildet ist.

18. Vorrichtung nach einem der Ansprüche 7 und 15, **dadurch gekennzeichnet,** dass die jeweils an dem Körper (35;35') angeordnete Blendscheibe (50;50') derart an einem Achskörper (72;72') um dessen Längsachse drehbar gelagert ist, dass die in Umfangsrichtung orientiert an der Blendecheibe (50;50') angeordneten Abblendflächen (51.1 bis 51.6) beziehungsweise der in der Ausnehmung (52;52') der Blendscheibe (50;50') angeordnete Filter (60;60') zur Regulierung der Lichtintensität wahlweise in den zweiten Lichtkanal (L2;L2') einblendbar ist.

19. Vorrichtung nach den Ansprüchen 7 und 18, **dadurch gekennzeichnet**, dass die beiden Blendscheiben (50;50') jeweils über einen zugeordneten Antrieb (80;80') und damit wirkverbundener Einstellknöpfe (65;65') manuell oder mittels eines mit dem Antrieb (80;80) wirkverbundenen Elektromotors (M) einzeln oder miteinander um die Längsachse der Achskörper (72;72') in bezug auf die Abblendflächen (51.1 bis 51.6) beziehungsweise Filter (60;60') in bezug auf den zugeordneten Lichtkanal (L2;L2') einstellbar sind.

20. Vorrichtung nach Anspruch 19, **dadurch gekennzeichnet**, dass der mit den beiden Einstellknöpfen (65;65') oder mit dem Elektromotor (M) wirkverbundene Antrieb (80;80') als Zahnradgetriebe, beispielsweise als Stirnradgetriebe ausgebildet ist.

21. Vorrichtung nach einem der Ansprüche 7 bis 20, **dadurch gekennzeichnet**, dass die einzelnen Funktionselemente als eine eigenständige und zum Anschliessen der Beleuchtungssonde (10;10') ausgebildete Baueinheit ausgebildet sind, wobei die Baueinheit mindestens die Lichtquelle (40), den Körper (35; 35') mit den abgewinkelt zueinander angeordneten Lichtkanälen (L1,L2;L1',L2'), die darin angeordnete erste und zweite optische Linse (47,48;47',48'), das die Wärme auskoppelnde und die sichtbare Strahlung umlenkende Bauteil (43;43') sowie die wahlweise zur Regulierung der Lichtintensität mit den Abblendflächen (51.1 bis 51.6) beziehungsweise mit dem Filter (60;60') in den zweiten Lichtkanal (L2;L2') einschwenkbare Blendscheibe (50;50') umfasst.
